# EUROPÄISCHE PATENTANMELDUNG

(11) **EP 4 413 969 A1**
(43) Veröffentlichungstag der Anmeldung: **14.08.2024**
(21) Anmeldenummer: 23217943.2
(22) Anmeldetag: 19.12.2023
(51) Int. Cl.: A61K 8/04, A61K 8/06, A61K 8/11, A61K 8/14, A61K 8/44, A61K 8/49, A61K 8/92, A61Q 19/00, A61Q 19/08

(54) **DISPERSION UND ZUBEREITUNG MIT CINEOL ZUR TOPISCHEN ANWENDUNG**

(30) Priorität: 10.02.2023 DE 102023103292
(71) Anmelder: Cineolux GmbH, 40219 Düsseldorf (DE)
(72) Erfinder: Kühlmann, Britta Anna, 40219 Düsseldorf (DE)
(74) Vertreter: Kohlmann, Kai

(57) **Zusammenfassung**

Die Erfindung betrifft eine wässrige Dispersion zur kosmetischen Applikation mit darin dispergierten Primärteilchen mit einer Teilchengröße von 10 bis 1000 nm, wobei die Primärteilchen eine Lipidmatrix auf der Basis von bei 20°C festen Lipiden aufweisen und mindestens einen Wirkstoff enthalten. Um wirkungsrelevante Konzentrationen von Cineol als Wirkstoff kosmetisch zu applizieren, jedoch irritative und sensibilisierende Effekte weitgehend zu vermeiden, wird eine Verkapselung von Cineol mithilfe eines modifizierten Trägersystems auf der Basis von Solid Lipid Nanopartikeln (SLNs) mit verzögerter Freisetzung des Wirkstoffs Cineol vorgeschlagen. Außerdem betrifft die Erfindung eine Zubereitung zur topischen Anwendung auf der Haut eines menschlichen Körpers.

## Beschreibung

Die Erfindung betrifft eine wässrige Dispersion zur kosmetischen Applikation mit darin dispergierten Primärteilchen mit einer Teilchengröße von 10 bis 1000 nm, wobei die Primärteilchen eine Lipidmatrix auf der Basis von bei 20°C festen Lipiden aufweisen und mindestens einen Wirkstoff enthalten.

Außerdem betrifft die Erfindung eine Zubereitung zur topischen Anwendung auf der Haut eines menschlichen Körpers.

Nanodispergierte Systeme, wie Lipid Nanopartikel, haben in den letzten Jahren als potentielle Vehikel für die kontrollierte Abgabe von Wirkstoffen an bestimmte Hautschichten Bedeutung erlangt. Solid Lipid Nanopartikel stellen dabei ein alternatives Trägersystem zu traditionellen kolloidalen Trägersystemen, wie Emulsionen, Liposomen und polymerbasierten Micro- oder Nanopartikeln, dar.

Derartige Trägersysteme, insbesondere in Form von Solid Lipid Nanopartikeln (SLN) oder nanostrukturierten Lipidträgern (NLC) sind an sich aus dem Stand der Technik bekannt. Beispielhaft für im Stand der Technik beschriebene SLNs und NLCs wird auf die WO 2002/051390 A1, WO 2001/003652 A1, DE 4131562 A1, EP 2 658 610 B1, DE 199 38 371 A1 und DE 199 38 371 A1 hingewiesen.

Solid Lipid Nanopartikel (SLN) wurden zu Beginn der 1990er Jahre entwickelt und entstehen durch das Ersetzen eines flüssigen Öls in einer Öl-in-Wasser-Emulsion durch ein festes Lipid oder ein Gemisch fester Lipide. Die festen Lipide sind sowohl bei Raumtemperatur als auch bei Körpertemperatur fest.

SLNs bestehen in der Regel aus 0,1 - 30% (w/w) des festen Lipids, das in wässrigem Medium durch 0,5 bis 5% (w/w) Emulgator stabilisiert wird. Der durchschnittliche Partikeldurchmesser von SLNs liegt im Bereich von 40 bis 1000 nm.

Eine Weiterentwicklung von festen Lipid Nanopartikeln stellen die sogenannten nanostrukturierten Lipidträger (NLCs) dar, die aus Mischungen von festen Lipiden und flüssigen Lipiden (Ölen) in einem Verhältnis von 70:30 bis 99,9:0,1 bestehen. Nanostrukturierte Lipidträger weisen aufgrund dieser Lipidmischungen im Vergleich zu den aus ausschließlich festen Lipiden basierenden Nanopartikeln einen geringeren Schmelzpunkt auf, sind jedoch genau wie diese bei Körpertemperatur (37°C) fest. Im Vergleich zu SLNs weisen NLCs für eine Reihe von Wirkstoffen eine höhere Beladungskapazität auf, erlauben die Formulierung von Partikelsuspensionen mit geringerem Wassergehalt und zeigen eine verminderte Abgabe des Wirkstoffs während der Lagerung.

1,8-Cineol gehört zu den bicyclischen Epoxy-Monoterpenen, genauer den Limonenoxiden. Die organische Verbindung liegt bei Raumtemperatur als farblose Flüssigkeit vor. 1,8-Cineol kommt in größeren Mengen in Eukalyptus, Arznei-Engelwurz (Angelica archangelica), Rosmarin (Rosmarinus officinalis), Salbei (Salvia officinalis ), Thai-Ingwer (Alpinia galanga), Majoran (Origanum majorana) und Lorbeer vor.

Der Wirkstoff 1,8-Cineol ist expektorationsfördernd, sekretomotorisch, schwach hyperämisierend und lokalanästhesierend. Es verflüssigt zähen Schleim und führt dadurch zu einer Erleichterung des Abhustens sowie zu einer Besserung der Symptome bei akuter Rhinosinusitis. In-vitro wurde neben einer antiviralen Wirkung auch eine antimikrobielle Wirkung gegen ein breites Spektrum von Grampositiven und Gram-negativen Bakterien sowie gegen Pilze gezeigt.

1,8-Cineol findet Anwendung bei der symptomatischen Behandlung von Atemwegserkrankungen wie Bronchitis bzw. Erkältungskrankheiten der Atemwege, aber auch als begleitende Behandlung von chronischen und entzündlichen Atemwegserkrankungen sowie Asthma und Heuschnupfen. Außerdem hemmt es bestimmte Neurotransmitter, die für die Verengung der Bronchien verantwortlich sind. Bei Asthmatikern kann unter ärztlicher Kontrolle durch Gabe von reinem Cineol die Lungenfunktion verbessert werden. Auch bei der chronischobstruktiven Lungenerkrankung COPD kann reines Cineol als Zusatzmedikation zur Standardtherapie unter Umständen die Lungenfunktion verbessern.

Die Einnahme erfolgt durch orale Zufuhr von Kapseln, die sich erst im Dünndarm auflösen, durch Inhalation oder durch Zubereitung entsprechender den Wirkstoff enthaltender Pflanzen als Aufguss.

Eine unmittelbare kosmetische bzw. topische Anwendung des Wirkstoffs Cineol ist nach Kenntnis des Anmelders bisher nicht erfolgreich erfolgt, da Cineol irritativ und sensibilisierend, insbesondere bei hohen Konzentrationen ist.

Ausgehend von diesem Stand der Technik liegt der Erfindung die Aufgabe zugrunde, eine wässrige Dispersion der eingangs erwähnten Art zu schaffen, mit der sich wirkungsrelevante Konzentrationen von Cineol kosmetisch applizieren lassen, jedoch irritative und sensibilisierende Effekte weitgehend vermieden werden. Außerdem soll eine Zubereitung unter Verwendung der wässrigen Dispersion geschaffen werden.

Die Lösung dieser Aufgabe basiert auf der Verkapselung von Cineol als Wirkstoff mithilfe eines modifizierten Trägersystems auf der Basis von Solid Lipid Nanopartikeln (SLNs) mit verzögerter Freisetzung des Wirkstoffs Cineol.

Im Einzelnen wird die Aufgabe durch eine wässrige Dispersion der eingangs erwähnten Art gelöst, wobei
- die Lipidmatrix mindestens zwei Lipide mit unterschiedlicher Schmelztemperatur aufweist, die derart bestimmt und gemischt werden, dass die Mischung der Lipide eine Schmelztemperatur im Bereich zwischen 35 - 40°C, bevorzugt im Bereich zwischen 37° - 39°C aufweist,
- der mindestens eine Wirkstoff 1,8-Cineol ist,
- die Menge des 1,8-Cineol zwischen 5 Gew.% - 20 Gew.% der Dispersion beträgt,
- die Menge der mindestens zwei Lipide zwischen 5 - 40 Gew.% der Dispersion beträgt und
- die Dispersion mindestens einen Emulgator enthält

Abweichend zu den bekannten Trägersysteme auf der Basis von Solid Lipid Nanopartikeln (SLN) ist das Gemisch der festen Lipide lediglich bei Raumtemperatur (20 °C) im festen Aggregatzustand, während die Mischung bei Körpertemperatur im Bereich zwischen 37° - 39°C bereits die Schmelztemperatur erreicht.

Die Einstellung der Schmelztemperatur beruht auf der Nutzung des Prinzips der Schmelzpunkterniedrigung von Mischungen, auch als Schmelzpunktdepression bezeichnet. Durch gezielte Auswahl der Lipide und Bestimmung von deren Mengen in der Mischung wird die Schmelztemperatur in dem Bereich zwischen 35°C - 40°C, vorzugsweise in einem Bereich zwischen 37° - 39°C eingestellt.

Besonders gute Ergebnisse konnten beispielsweise mit einer Mischung aus den Lipiden Bienenwachs (Cera Alba) und Carnaubawachs (Copernica Cerifera Cera) erzielt werden. Obwohl Bienenwachs allein erst bei Temperaturen über 60° und Carnaubawachs erst über 80 °C schmilzt, liegt die Schmelztemperatur der Mischung im Bereich der Körpertemperatur zwischen 37 °C bis 39 °C, wenn das Massenverhältnis von Carnaubawachs und Bienenwachs etwa 1,8:1 beträgt.

Erfindungsgemäß kommen vorzugsweise anionische Tenside als Emulgatoren zum Einsatz. Als anionische Tenside bezeichnet man Tenside, die eine negativ geladene funktionelle Gruppe besitzen. Als unpolarer Teil dient ein Alkylrest. Die polare funktionelle Gruppe ist -COO⁻(Carboxylat), - SO₃⁻(Sulfonat) oder -SO₄²⁻(Sulfat) .

Die anionischen Tenside dienen der Aufrechterhaltung der Nanokristallsuspension; sie können ein ausreichend hohes Zeta-Potential erzeugen, damit die elektrostatische Abstoßung größer als die Gravitationskraft der kleinen Kristalle ist. Folgende Substanzklassen anionischer Tenside kommen in Frage:
- Alkylcarboxylate
- Alkylbenzolsulfonate
- sekundäre Alkylsulfonate (auch Alkansulfonate, SAS): CnH2n+1-SO3-Na+, auch fluoriert, zum Beispiel Kalium-N-ethyl-N-((heptadecafluoroctyl)sulfonyl)glycinat
- Fettalkoholsulfate
- Alkylethersulfate,
- Sulfoacetate

Die anionischen Tenside bestehen aus einem hydrophoben (wasserabweisenden) und einem hydrophilen (wasserliebenden) Molekülteil und sind daher amphiphil. Gibt man die anionischen Tenside in das Wasser der Dispersion, bilden diese ab einer kritischen Konzentration innerhalb des Wassers Aggregate. Dabei richten sich die Tensidmoleküle so aus, dass die hydrophoben Enden sich im Inneren der Primärteilchen bzw. Lipidpartikel sammeln und die hydrophilen Enden sich in Richtung des Wassers orientieren. Die anionischen Tenside lagern sich um die Primärteilchen an und hemmen das Zusammenklumpen der Primärteilchen.

Ein Vorteil der Erfindung bestehet darin, dass der Wirkstoff Cineol aus der Lipidmatrix, die bei Körpertemperatur ihren Schmelzpunkt erreicht, zeitverzögert über einen längeren Zeitraum von mehreren Stunden, vorzugweise etwa 8 - 15 Stunden, abgegeben werden kann. Durch die retardierte Abgabe der Wirkstoffkomponente bei Körpertemperatur werden Irritationen der Hautoberfläche und Geruchsbelästigungen durch Cineol selbst bei hohen, wirkungsrelevanten Konzentration oberhalb von 5 Gew.%, vorzugsweise von 10 Gew.% in Bezug auf die Dispersion vermieden.

Es konnte gezeigt werden, dass die Applikation von 1,8-Cineol auf der Haut die Hautalterung verlangsamt. Durch u.a. RNA-Sequenz-Analysen menschlicher Hautproben konnte festgestellt werden, dass ein spezifisches Protein des WNT-Signalweges maßgeblich bei der Entstehung von Hautalterung involviert ist. Laborchemische und Kleintiermodell-Versuche haben bestätigt, dass 1,8-Cineol die Kollagensynthese angeregt und somit die Wundheilung beschleunigt und Hautalterung verzögert wird. Als natürlicher freier Radikalfänger ist dieser Wirkstoff ein potentes Antioxidans, welcher zu den bicyclischen Epoxy-Monoterpenen gehört. Zudem hat sich herausgestellt, dass 1,8-Cineol anti-entzündlich wirkt und vor UV-Schäden sowie oxidativem Stress der Haut schützen kann. Die hauteigene Ceramid-Produktion wird angeregt und die Barrierefunktion der Haut verbessert.

Bei Anwendung des Wirkstoffs in einer Zubereitung, z.B. als Creme, ist dieser antagonistische Wirkstoff der Schlüssel zu natürlicher Regeneration und verbesserter Funktionalität der Haut. Durch die modifizierte SLN-Verkapselung wird der Wirkstoff 1,8-Cineol in tieferliegende Hautschichten transportiert und wirkt effektiv am Zielort. Insbesondere werden durch die Verkapselung die bei topischer Anwendung ungünstigen Eigenschaften des Cineols, selbst bei relativ hohen Konzentrationen, abgemildert, was insbesondere durch die zeitverzögerte Freisetzung der Wirkstoffe bewirkt wird.

Um die wässrige Dispersion zu stabilisieren, weist diese vorzugsweise ein Zetapotential (bestimmt nach der Messmethode in Abhängigkeit vom pH-Wert mit dem Gerät Zetasizernano ZS, Malvern Instruments) größer 60 emV, vorzugsweise größer 75 emV auf.

Hinsichtlich der als Lipidmatrix zu verwendenden Lipide unterliegt die wässrige Dispersion gemäß der vorliegenden Erfindung folgenden Beschränkungen:
- Die mindestens zwei Lipide der Lipidmatrix liegen bei Raumtemperatur (20 °C) in fester Form vor.
- Die mindestens zwei Lipide der Lipidmatrix werden derart bestimmt und gemischt, dass die Mischung eine Schmelztemperatur im Bereich der Körpertemperatur (37 - 39 °C) aufweist.

Bevorzugt sind die festen Lipide ausgewählt aus der Gruppe bestehend aus Fettalkoholen, insbesondere Cethylalkohol, Fettsäureestern, insbesondere Partialfettsäureestern, insbesondere in Form von Cetylpalmitat, Isopropylpermitat, oder Octylpalmitat, Fettsäuren (z.B. Stearinsäure), Steroiden (z.B. Cholesterol), Terpenen, Saponinen, Wachsen, natürlich und/oder derivatisiert, insbesondere in Form von Bienenwachs und Carnaubawachs, Cetylpalmitat, Reiswachse, Beerenwachse (z.B. Rhus Verniciflua) -einzeln oder in Mischung, veresterten Proteinen, insbesondere Ceramiden (I und II) und/oder Phytosterole, dies sowohl natürlich als auch derivatisiert, (hoch-gereinigte) Triglyceride (z.B. Tristearin (Dynasan 118), Dynasan 114 (Trimyristin), Dynasan 116, Dynasan 112), Triolein, Glycerol, partielle Glyceride (z.B. Imwitor), Komplex-Glycerid Mischungen (z.B. Glyceryldistearat (Precirol ATO)), Glyceroldibehenat (z.B. Compritol 888 ATO), Glycerolmonostearat (z.B. Monostearin), Glyceryldistearat (z.B. Precirol ATO 5), langkettige Trigylceride (LCT), Isopropylmyristat, Paraffinlipide, Hartparaffine, Harzparaffine, mittelkettige Triglyceride (MCT), (z.B. Miglyol in unterschiedlichen Konzentrationen, z.B. Miglyol 812, Miglyol 810, Miglyol 840), pflanzliche Öle, wie Avocadoöl, Baumwollsamenöl, Distelöl, Erdnussöl, Jojobaöl, Kokosnussöl/Kokosnussfett, Leinöl, Nussöl, Olivenöl, Palmkernöl, Sesamöl, hydriertes Palmöl, Weizenkeimöl, tierische Öle, wie Lebertran, Heilbuttleberöl, Rinderklauenöl, Kakaobutter, Q10, Cetylpalmitat, Palmitinsäure, PEG 2000.

Der Vollständigkeit-halber wird noch auf sogenannte Repellents hingewiesen. Beispiele für Repellents sind natürliche Repellents wie Citrusöle, Eukalyptusöl und Campher oder synthetische Repellents, wie N,N-Diethyltoluamid (DEET), Dibutylphthalat, Dimethylphthalat, 2-Ethyl-1,3-hexandiol. Die Repellents können als Lipidpartikel alleine oder in Kombination mit Duftstoffen und/oder UV-Blockern vorliegen.

Die Dispersion kann in vorteilhafter Ausgestaltung der Erfindung mindestens einen zusätzlichen Wirkstoff aufweisen, der in den Primärteilchen immobilisiert ist. Der zusätzliche Wirkstoff kann vollständig oder nur teilweise in den Primärteilchen immobilisiert sein, wobei der Rest des zusätzlichen Wirkstoffs in der Dispersion vorliegt.

Bei den anwendungsspezifischen zusätzlichen Wirkstoffen handelt es sich insbesondere um Nicotinamid, Phytosterole aus dem Granatapfel, Phytosterole aus der Acaibeere, Lipoaminosäuren, Peptidestern, synthetische Ceramide, Ceramide, insbesondere Ceramide 3, lipophile/lipophilisierte Vitamine, Retinol, Tocopheron, Ubichinon, Ascorbyl-Palmitat, Hyaluronsäure, Hormone, Kollagen, Peptide, Antioxidanzien, Q10 (Coenzym Q10, Ubichinon-10), Azelainsäure (Azelaic Acid), Grüner Tee (Camellia Sinensis Leaf Extract), Kamille (Chamomilla Recutita Oil), Beta Glucan, Resveratrol, Astaxanthin, Alpha-hydroxy acids (AHA), Beta hydroxy acids (salicylic acid), Hydroquinone, Kojic acid, Acetylsalicylsäure, Squalen, Lipide und Feuchtigkeitsspender, biomimetische (=synthetische) Wirkstoffe und Peptide, z.B. Gluthation, Palmitoyl Pentapeptide-4, Dipeptide Diaminobutyroyl Benzylamide Diacetate, Agireline (INCI-Bezeichnung: Acetyl-Hexapeptide 3) sowie Oligopeptide wie z.B. Soja-Oligopeptide und hydrolysiertes Milchprotein, Cytokine und Wachstumsfaktoren, wie z.B. Fibroblast Growth Factor (FGF), Epidermal Growth Factor (EGF), Metformin, Pflanzenextrakt und Coenzyme, wie Nicotinamidadenindinukleotid (NAD), NAD+, NADH, Lipide oder Gemische davon.

Zu den anwendungsspezifischen zusätzlichen Wirkstoffen gehören darüber hinaus Vitamine und Pro-Vitamine. Zu den antioxidativen Vitaminen gehören Vitamin C (Ascorbinsäure, Ethyl Ascorbic Acid, Ascorbylpalmitat, Tetrahexyldecyl-Ascorba), Vitamin E (Tocopherol) und Vitamin A (Retinol, Retinol-Ester (Retinyl Palmitate, Retinyl Linoleate). Als Vitamin kommen Linolsäure/alpha-Linolsäure (Vitamin F) in Betracht.

Als besonders vorteilhaft hat sich der Zusatz von Nicotinamid herausgestellt. Nicotinamid ist ein wasserlösliches Vitamin und gehört zur Gruppe der B-Vitamine. Es spielt im Körper durch seine Beteiligung an vielen enzymatischen Vorgängen eine wichtige Rolle. Nicotinamid ist an der Verstoffwechselung von Eiweißen, Fetten und Kohlenhydraten beteiligt. Insbesondere unterstützt das Vitamin B3 die Regeneration der Haut im Zusammenwirken mit dem 1,8-Cineol. Daneben wird Nicotinamid ebenfalls eine antioxidative, die Hautalterung günstig beeinflussende Wirkung zugeschrieben.

Bevorzugte Feuchtigkeitsspender sind: Hyaluronsäure (Hyaluronic Acid, Sodium Hyaluronate), Urea (Harnstoff), Glycerin, Milchsäure (Lactic Acid), Glycolsäure (Glycolic Acid), Pentylenegylcol, Butyleneglycol, Panthenol.

Darüber hinaus ist es vorteilhaft, wenn die Primärteilchen zusätzliche Additive in Form von Antioxidationsmitteln, insbesondere Tocopherole, Ascorbyl-Palmitat, Retinol und Derivate hiervon, acylierte Polysaccharide, Feuchthaltemittel, wie Pentylenglycol oder Caprilylglycol, Sulfur, Titandioxid, Emulgatoren und/oder Konservierungsmittel, insbesondere in Form von Phenoxyethanol oder Etylhexylglycerin, enthalten. Es hat sich als zweckmäßig erwiesen wenn die Primärteilchen einen Gehalt an solchen Additiven von bis zu etwa 5 Gew.-%, insbesondere von bis zu etwa 1 Gew.-% bezogen auf die Primärteilchen aufweisen.

Um die wirkungsrelevante Mengen an 1,8- Cineol in die Primärteilchen einzulagern, befinden sich die bei 20 °C festen Lipide in einem kristallinen Zustand mit gestörter Gitterstruktur.

Für die kosmetische Applikation der erfindungsgemäßen wässrigen Dispersion ist es bevorzugt, wenn die nanoskaligen Primärteilchen eine Teilchengröße von 150 bis 220 nm aufweisen.

Die Menge der Lipide bezogen auf die wässrige Dispersion, liegt in der Regel im Bereich von 5 bis 45 Gew.-%, bevorzugt im Bereich von 10 bis 35 Gew.-%, und insbesondere im Bereich zwischen 10 und 25 Gew.-%.

Eine Zubereitung zur topischen Anwendung auf der Haut umfasst die wässrige Dispersion, die einer Creme als Grundlage der Zubereitung zugemischt ist. Als Grundlage der Zubereitung kommen insbesondere amphiphile Cremes, z.B. Basiscreme DAC, hydrophile Creme, z.B. anionische hydrophile Creme DAC, hydrophile Lotion, z.B. hydrophile Basisemulsion DAC oder hydrophiles Gel, z.B. Hydroxyethylcellulosegel DAB in Betracht.

Die Zubereitung hat einen Okklusionseffekt; dieser tritt auf, weil die Hautoberfläche mit lipophilen Stoffen abgedeckt wird. Hierdurch wird der Austritt von Flüssigkeit aus der Haut reduziert, was ein Flüssigkeitsstau im Gewebe zur Folge hat und dadurch die Diffusion des Wirkstoffs Cineol verbessert.

Nachfolgend wird die Zusammensetzung eines bevorzugten Ausführungsbeispiels der Dispersion angegeben:

| **Rohstoff** | **Menge in Gew. %** |
|---|---|
| Phase I | |
| 1,8-Cineol | 10,00 |

| Phase II | |
|---|---|
| Cera Alba (Bienenwachs) | 2,75 |
| Copernica Cerifera Cera (Carnaubawachs) | 5,00 |

| Phase III | |
|---|---|
| dem. Aqua | 78,25 |
| Konservierungsmittel: | 1,00 |
| (Phenoxyethanol + Ethyhexyglycerin) | (0,9 + 0,1) |
| Anionischer Emulgator: | 3,00 |
| Sodium Stearoyl Glutamate | |
| | **100** |

Die wässrige Dispersion umfasst 10 Gew.-% des Wirkstoffs 1,8-Cineol bezogen auf die Menge der Dispersion, ein Gemisch fester Lipide, das zusammen 7,75 Gew.% der Dispersion beträgt, wobei die festen Lipide in dem wässrigem Medium durch einen anionisches Tensid als Emulgator stabilisiert werden.

AMISOFT^{®} HS-11 (Sodium Stearoyl Glutamate) ist ein geeignetes anionisches Tensid in Pulverform. Hergestellt wird Amisoft HS-11 aus L-Glutaminsäure und pflanzlicher Palmfettsäure. Sein Anwendungs-pH ähnelt dem der Haut. Es hat relativ geringe Reinigungs- und Schaumeigenschaften. Der Emulgator dient dem Zweck, der Aggregationsneigung der Fettkristalle durch gleichnamige Ladungen entgegenzuwirken.

Bei den festen Lipidkomponenten handelt es sich in dem Ausführungsbeispiel um Carnaubawachs und Bienenwachs:
Carnaubawachs, auch Brasilianisches Wachs oder Cearawachs, stammt aus dem Blatt-Exsudat der in Brasilien wachsenden Carnaubapalme (Copernicia Cerifera).

Bienenwachs (Cera Alba) ist ein von Honigbienen abgesondertes Wachs, das von ihnen zum Bau der Bienenwaben genutzt wird. Bienenwachs wird in der kosmetischen und pharmazeutischen Industrie als Bestandteil u.a. von Cremes, Salben und Lotionen verwendet.

Funktional sind die Kristalle aus den beiden natürlichen Wachsen derart eingestellt, dass sie bei Raumtemperatur fest sind und der Schmelzpunkt der Körpertemperatur (etwa 37 Grad) entspricht, wobei eine Freisetzungsdauer des Wirkstoffs 1,8-Cineol über einen Zeitraum von etwa 12 Stunden angestrebt ist. Das Sintern der Kristalle beginnt ab etwa 36 °C

Microcare^{®} PEHG ist ein flüssiges Konservierungsmittel auf der Basis der Kombination von Phenoxyethanol und Ethylhexylglycerin.

Weitere Versuche lassen erwarten, dass sich der Anteil des 1,8-Cineol in dem Trägersystem auf bis zu maximal 20 Gew.% steigern lässt.

Die wässrige Dispersion wird für eine topische Anwendung auf der Haut mit etwa 10% Gew.% in eine Zubereitung in Form einer Creme eingearbeitet, um die angestrebte Anwendungskonzentration von 1,8- Cineol zu erreichen.

Für die Herstellung der Primärteilchen können verschiedene Verfahren angewandt werden, insbesondere jedoch die Hochdruckhomogenisierung. Bei der Hochdruckhomogenisierung können zwei Verfahren unterschieden werden, die als kalte Hochdruckhomogenisierung oder heiße Hochdruckhomogenisierung bezeichnet werden.

Bei der kalten Hochdruckhomogenisierung wird der Wirkstoff 1,8-Cineol zunächst in eine Schmelze der festen Lipide eingemischt, die abkühlt und im festen Zustand zu Lipid-Micropartikeln vermahlen wird. Diese werden in einer kalten Emulgator Lösung suspendiert und im Hochdruckhomogenisator zu Nanopartikeln verarbeitet.

Bei der heißen Hochdruckhomogenisierung wird der Wirkstoff 1,8-Cineol ebenfalls zunächst in einer Schmelze des festen Lipids dispergiert und anschließend in einer heißen Emulgator Lösung dispergiert. Die erhaltene Dispersion wird dann ebenfalls im Hochdruckhomogenisator in eine Nanopartikel-Dispersion umgewandelt, in der sich die Nanopartikel beim anschließenden Abkühlen verfestigen.

## Patentansprüche

1. Wässrige Dispersion zur kosmetischen Applikation mit darin dispergierten Primärteilchen (Lipidpartikeln) mit einer Teilchengröße von 10 bis 1000 nm, wobei die Primärteilchen eine Lipidmatrix auf der Basis von bei 20°C festen Lipiden aufweisen und mindestens einen Wirkstoff enthalten, **dadurch gekennzeichnet, dass**
- der mindestens eine Wirkstoff 1,8-Cineol ist,
- die Lipidmatrix mindestens zwei Lipide mit unterschiedlicher Schmelztemperatur aufweist, die derart bestimmt und gemischt werden, dass die Mischung der Lipide eine Schmelztemperatur im Bereich zwischen 35°C - 40°C aufweist,
- die Menge des 1,8-Cineol zwischen 5 Gew.% - 20 Gew.% der Dispersion beträgt,
- die Menge der mindestens zwei Lipide zwischen 5 - 45 Gew.% der Dispersion beträgt und
- die Dispersion Tenside als Emulgator enthält.

2. Wässrige Dispersion nach Anspruch 1, **dadurch gekennzeichnet, dass** das Zetapotential der Dispersion (bestimmt nach der Messmethode in Abhängigkeit vom pH-Wert mit dem Gerät Zetasizer Nano ZS, Malvern Instruments) größer 60 emV, vorzugsweise größer 75 emV ist.

3. Wässrige Dispersion nach Anspruch 1 oder 2, **dadurch gekennzeichnet, dass** die festen Lipide ausgewählt sind aus der Gruppe umfassend Fettalkohole, Fettsäureestern, Fettsäuren, Steroide, Terpene, Saponine, Wachse, natürlich und/oder derivatisiert, einzeln oder in Mischung, veresterten Proteinen, insbesondere Ceramiden (I und II) und/oder Phytosterole, dies sowohl natürlich als auch derivatisiert, Triglyceride, Triolein, Glycerol, partielle Glyceride, Komplex-Glycerid Mischungen, Glycerolmonostearat, Glyceryldistearat, langkettige Trigylceride (LCT), Isopropylmyristat, Paraffinlipide, Hartparaffine, Harzparaffine, mittelkettige Triglyceride (MCT), pflanzliche Öle, tierische Öle, Kakaobutter, Q10, Cetylpalmitat, Palmitinsäure, PEG 2000.

4. Wässrige Dispersion nach einem der Ansprüche 1 bis 3, **dadurch gekennzeichnet, dass** die Primärteilchen zusätzliche anwendungsspezifische Wirkstoffe für die Haut enthalten.

5. Wässrige Dispersion nach Anspruch 4, **dadurch gekennzeichnet, dass** die zusätzlichen Wirkstoffe aus der Gruppe ausgewählt sind, umfassend Nicotinamid, Phytosterole aus dem Granatapfel, Phytosterole aus der Acaibeere, Lipoaminosäuren, Peptidestern, synthetische Ceramide, Ceramide, insbesondere Ceramide 3, lipophile/lipophilisierte Vitamine, Retinol, Tocopheron, Ubichinon, Ascorbyl-Palmitat, Hyaluronsäure, Hormone, Kollagen, Peptide, Antioxidanzien, Q10 (Coenzym Q10, Ubichinon-10), Azelainsäure (Azelaic Acid), Grüner Tee (Camellia Sinensis Leaf Extract), Kamille (Chamomilla Recutita Oil), Beta Glucan, Resveratrol, Astaxanthin, Alpha-hydroxy acids (AHA), Beta hydroxy acids (salicylic acid), Hydroquinone, Kojic acid, Acetylsalicylsäure, Squalen, Lipide und Feuchtigkeitsspender oder Gemische davon.

6. Wässrige Dispersion nach einem der Ansprüche 1 bis 5, **dadurch gekennzeichnet, dass** die Primärteilchen zusätzliche Additive in Form von Antioxidationsmitteln, insbesondere Tocopherole, Ascorbyl-Palmitat, Retinol und Derivate hiervon, acylierte Polysaccharide, Feuchthaltemittel, wie Pentylenglycol oder Caprilylglycol, Sulfur, Titandioxid, Emulgatoren und/oder Konservierungsmittel, insbesondere in Form von Phenoxyethanol oder Etylhexylglycerin, enthalten.

7. Wässrige Dispersion nach einem der Ansprüche 1 bis 6, **dadurch gekennzeichnet, dass** die bei 20°C festen Lipide sich in einem kristallinem Zustand mit gestörter Gitterstruktur befinden.

8. Wässrige Dispersion nach einem der Ansprüche 1 bis 7, **dadurch gekennzeichnet, dass** die Primärteilchen eine Teilchengröße von 150 bis 220 nm aufweisen.

9. Wässrige Dispersion nach einem der Ansprüche 1 bis 8, **dadurch gekennzeichnet, dass** die Menge der mindestens zwei Lipide zwischen 5 - 35 Gew.% der Dispersion beträgt.

10. Wässrige Dispersion nach einem der Ansprüche 1 bis 9, **dadurch gekennzeichnet, dass** die Menge des Emulgators mindestens 0,5 Gew.% und höchstens 5 Gew.% der Dispersion beträgt.

11. Wässrige Dispersion nach einem der Ansprüche 1 bis 11, **dadurch gekennzeichnet, dass** die Emulgatoren anionische Tenside sind.

12. Wässrige Dispersion nach Anspruch 11, **dadurch gekennzeichnet, dass** die Emulgatoren aus der Gruppe ausgewählt sind, die folgende Substanzklassen umfasst:
• Alkylbenzolsulfonate
• sekundäre Alkylsulfonate (auch Alkansulfonate, SAS): CnH2n+1-SO3-Na+, auch fluoriert, zum Beispiel Kalium-N-ethyl-N-((heptadecafluoroctyl)sulfonyl)glycinat
• Fettalkoholsulfate
• Alkylethersulfate,
• Sulfoacetate

13. Zubereitung zur topischen Anwendung auf der Haut, **dadurch gekennzeichnet, dass** die wässrige Dispersion nach einem oder mehreren der Ansprüche 1 bis 12 in eine Creme eingearbeitet ist.

14. Zubereitung nach Anspruch 10, **dadurch gekennzeichnet, dass** die Menge der wässrigen Dispersion mindestens 10 Gew.% der Zubereitung beträgt.
